(19) Europäisches Patentamt
European Patent Office
Office européen des brevets

(11) EP 1 226 809 B1

(12) **EUROPEAN PATENT SPECIFICATION**

(45) Date of publication and mention of the grant of the patent:
**30.05.2007 Bulletin 2007/22**

(21) Application number: **00970168.1**

(22) Date of filing: **27.10.2000**

(51) Int Cl.:
***A61K 8/49*** *(2006.01)* ***A61Q 9/00*** *(2006.01)*

(86) International application number:
**PCT/JP2000/007585**

(87) International publication number:
**WO 2001/032131 (10.05.2001 Gazette 2001/19)**

(54) **SKIN TEXTURE-IMPROVING AGENTS**

MITTEL ZUR VERBESSERUNG DER HAUTTEXTUR

AGENTS D'AMELIORATION DE TEXTURE DE LA PEAU

(84) Designated Contracting States:
**AT BE CH CY DE DK ES FI FR GB GR IE IT LI LU MC NL PT SE**

(30) Priority: **29.10.1999 JP 31013799**

(43) Date of publication of application:
**31.07.2002 Bulletin 2002/31**

(73) Proprietor: **KYOWA HAKKO KOGYO CO., LTD.**
**Chiyoda-ku,**
**Tokyo 100-8185 (JP)**

(72) Inventors:
- **TAKAHASHI, Tomoya,**
**Kyowa Hakko Kogyo Co., Ltd.**
**Tsukuba-shi,**
**Ibaraki 305-0841 (JP)**
- **TAKABOSHI, Chiemi,Kyowa Hakko Kogyo Co., Ltd.**
**Tsukuba -shi,**
**Ibaraki 305-0841 (JP)**
- **KOBAYASHI, Asako,Kyowa Hakko Kogyo Co., Ltd.**
**Tsukuba- shi,**
**Ibaraki 305-0841 (JP)**
- **TAJIMA, Minako,**
**Kyowa Hakko Kogyo Co., Ltd.**
**Tsukuba-shi,**
**Ibaraki 305-0841 (JP)**
- **KAMIMURA, Ayako,**
**Kyowa Hakko Kogyo Co., Ltd.**
**Tsukuba-shi,**
**Ibaraki 305-0841 (JP)**

(74) Representative: **Harding, Charles Thomas et al**
**D Young & Co**
**120 Holborn**
**London EC1N 2DY (GB)**

(56) References cited:

| | |
|---|---|
| **EP-A- 1 025 835** | **WO-A-01/26617** |
| **WO-A-99/66881** | **JP-A- 2 134 309** |
| **JP-A- 6 336 418** | **JP-A- 6 336 419** |
| **JP-A- 6 336 421** | **JP-A- 6 336 422** |
| **JP-A- 6 336 423** | **JP-A- 6 336 430** |
| **JP-A- 7 233 046** | **JP-A- 10 236 943** |
| **JP-A- 11 246 562** | **JP-A- 2000 290 124** |
| **US-A- 5 804 594** | |

- **"OPC Proanthocyanidin" WELLNESS ADVOCATE, vol. 4, no. 9, September 1994 (1994-09), pages 1-4, XP002223076**
- **MASQUELIER J.: "Oligomères procyanidoliques" PARFUMS, COSM[1]TIQUES & AROMES, no. 95, October 1990 (1990-10) - November 1990 (1990-11), XP000165236**
- **MASQUELIER J.: "OPC oligomères procyanidoliques" SOFW-JOURNAL SEIFEN, OELE, FETTE, WACHSE, vol. 117, no. 2, 1991, page 55 XP000147195 VERLAG FUR CHEMISCHE INDUSTRIE, H. ZIOLKOWSKY K.G. AUGSBURG, DE**
- **PLUM G.W. ET AL.: "Antioxidant Properties of Catechins and Proanthocyanidins: Effect of Polymerisation, Galloylation and Glycosylation" FREE RAD. RES., vol. 29, 1998, pages 351-358, XP001080646**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 226 809 B1

## Description

### Technical Field

The present invention relates to the cosmetic use of a composition comprising, as an active ingredient, proanthocyanidin in which the extent of galloylation per constitutive monomer is 10 % or lower (molar ratio) for increasing skin water-content or promoting epidermal ceramide synthesis.

### Background Art

The normal functioning of the skin horny layer is important for the skin to keep its moisture and softness. The horny layer has both the ability to keep moisture and the barrier function to prevent evaporation of moisture and invasion by foreign substances from the outside. However, it is known that the ability to keep moisture or the barrier function deteriorates by internal factors such as aging or allergic diseases or external factors such as ultraviolet rays, which causes damage to the skin in respect of moisture and softness.

The known factors related to the retention of moisture in the horny layer are components of natural moisturizing factor (NMF), sebum components and intercellular lipids in the horny layer. The NMF components are concerned in the absorption and retention of moisture in the skin and the sebum components and intercellular lipids in the horny layer are concerned in the barrier function.

Known examples of the NMF components include free amino acids such as serine, pyrrolidonecarboxylic acid, lactates, urea, inorganic salts and organic acids. Known examples of the sebum components include cholesterol esters, triglycerides, squalene, wax, free fatty acids, free cholesterol, ceramide and cholesteryl sulfate. Known examples of the intercellular lipids in the horny layer include cholesterol, ceramide, glycosyl ceramide and sphingolipids [Fragrance Journal, Vol. 1, p. 71-80 (1995); *ibid.,* Vol. 5, p. 40-48 (1991)]. Application of these components to cosmetic formulae has so far been proposed. That is, NMF components, high molecular substances such as hyaluronic acid, or polyhydric alcohols such as glycerin, propylene glycol or 1,3-butylene glycol have been used as moisturizers, and various oil components have been used for the purpose of skin protection. However, there are few known substances which can exhibit the activity to improve the skin by enhancing the function of the horny layer. Allantoin, aloe extract, ginseng extract, placenta extract, bovine blood freed of protein, yeast fermentation metabolites, etc. have been employed to keep the skin moist and to improve the skin, but their effects are not sufficient.

Dermal extracellular matrix components such as dermal collagen are known as molecules concerned in the formation of skin wrinkles. The change in the dermal extracellular matrix is considered to be responsible for the formation of long, deep and distinct wrinkles. Collagen, which accounts for over 90% of the dermal extracellular matrix components, is assumed to be greatly concerned in the formation of wrinkles [Skin Science, Vol. 30 (No. 3), p. 5186-5190 (1998)]. It is also reported that concomitantly with the formation of wrinkles by irradiation with long wavelength ultraviolet rays (UVB; 320-400 nm), the collapse of the fascicle structure of collagen is observed [Fragrance Journal, Vol. 4, p. 36-42 (1998)], while the expression of collagenase related to the degradation system is promoted by irradiation with UVB or short wavelength ultraviolet rays (UVA; 290-320 nm) [Fragrance Journal, Vol. 4, p. 43-47 (1998)]. Thus, it is expected that the promotion of collagen synthesis and the inhibition of collagenase will inhibit the wrinkle formation and will contribute to the improvement of the skin.

On the other hand, it is known in the cosmetic field that proanthocyanidin is effective for the treatment of atopic dermatitis when used with coumarin (Japanese Published Unexamined Patent Application No. 59463/96) and for the improvement of rough skin when used with a mucopolysaccharide and/or a protein (Japanese Published Unexamined Patent Application No. 336423/94). As the agents for external application comprising proanthocyanidin as an active ingredient, a Maillard reaction inhibitor (Japanese Published Unexamined Patent Application No. 336430/94) and a whitening agent (Japanese Published Unexamined Patent Application No. 134309/90) are known.

### Disclosure of the invention

An object of the present invention is the use of a cosmetic composition as disclosed in claim 1.

As a result of intensive studies on the skin-improving activity, the present inventors have discovered a skin-improving effect in proanthocyanidin in which the extent of galloylation per constitutive monomer is 10% or lower (molar ratio) .

The present invention relates to the following.

(1) Cosmetic use of a composition comprising, as an active ingredient, proanthocyanidin in which the extent of galloylation per constitutive monomer is 10% or lower (molar ratio) for increasing a skin water content or promoting epidermal ceramide synthesis.

(2) Use according to the above (1), wherein the proanthocyanidin is not derived from pine bark.

(3) Use according to the above (1), wherein the proanthocyanidin is derived from apple.

(4) Use according to any of the above (1) to (3), wherein the proanthocyanidin comprises ungalloylated proanthocyanidin oligomers (dimers to 5-mers).

(5) Use according to any of the above (1) to (4), wherein the proanthocyanidin content is 0.01 to 10% (w/w).

(6) Use according to any of the above (1) to (5), wherein the proportion of ungalloylated proanthocyanidin oligomers which are dimers to the total proanthocyanidin is 30% or higher (w/w).

(7) Use according to any of the above (1) to (5), wherein the proportion of ungalloylated proanthocyanidin oligomers which are dimers or trimers to the total proanthocyanidin is 40% or higher (w/w).

(8) Use according to any of the above (1) to (5), wherein the proportion of ungalloylated proanthocyanidin oligomers which are dimers to 5-mers to the total proanthocyanidin is 50% or higher (w/w).

The proanthocyanidin to be used in the present invention includes, for example, condensed tannin contained in various plants. Condensed tannin is polymers comprised of flavanol skeletons. Specific examples are compounds in which flavan-7-ol derivatives as constitutive units are successively condensed or polymerized by bonds such as 40α→6, 4α→ 8, 4β→ 6, 4β→ 8 or 4β→8·2βO→7. Examples of the monomers which constitute proanthocyanidin for use herein are monomers of flavan-7-ol derivatives. Preferred are compounds represented by formula (I):

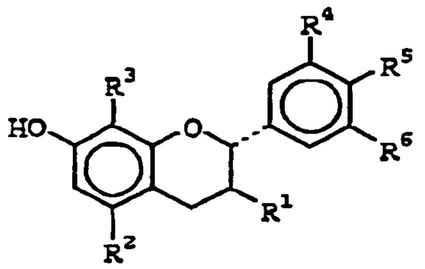 (I)

(wherein $R^1$, $R^2$, $R^3$, $R^4$, $R^5$ and $R^6$, which may be the same or different, each represents a hydrogen atom, a hydroxyl group or -O-galloyl).

Examples of the proanthocyanidin for use herein are procyanidin, prodelphinidin, propelargonidin, and isomers thereof.

There is no specific restriction as to the origin of the proanthocyanidin to be used in the present invention, but plant materials such as fruits, rinds, seeds or barks, extracts thereof or partially purified products thereof are preferred. For example, materials containing proanthocyanidin oligomers in large amounts such as fruit juice (e.g. apple juice or cowberry juice), extracts of rinds or seeds, inner coats of peanuts or chestnuts, barley husks, buckwheat chaff, or extracts of persimmon leaves, pine bark or coconuts are preferred. Specifically preferred are apple-derived proanthocyanidin oligomers which are known to be ungalloylated [Fragrance Journal, Vol. 4, p. 63-69 (1997)]. When persimmon fruits or grape seeds containing galloylated proanthocyanidin in large amounts are employed, ungalloylated proanthocyanidin in which the extent of galloylation per constitutive monomer is 10% or lower (molar ratio) can be obtained by treatment with tannase [Acta Dermato Venereologica, Vol. 78, p. 428-432 (1998)].

As for the method for the synthesis of ungalloylated proanthocyanidin oligomers, methods for the production of dimers of epicatechin or catechin are described in Journal of Chemical Society, Perkin Transaction I, p. 1535-1543 (1983), etc., and methods for the production of trimers of epicatechin or catechin are described in Phytochemistry, Vol. 25, p. 1209-1215 (1986) The ungalloylated proanthocyanidin oligomers to be used in the present invention can be obtained by these methods.

Extraction and purification of proanthocyanidin from plant materials can be carried out according to known methods.

Plant materials such as fruits, seeds, inner coats, shells, leaves or barks are used, as such or usually after being subjected to drying such as air drying, as materials for extraction. Extraction of proanthocyanidin from the materials for extraction can be carried out according to known methods, for example, the methods described in Chemical & Pharmaceutical Bulletin, Vol. 38, p. 3218-3225 (1990); *ibid.,* Vol. 40, p. 889-898 (1992) ., or a method similar thereto.

Juice or sap of plants may be used as such for extraction, or a concentrated extract of condensed tannin may be prepared by referring to Agricultural and Biological Chemistry, Vol. 45, p. 1885-1887 (1981).

Extraction of proanthocyanidin is usually carried out after the above plants are cut into fine pieces using a hydrophilic solvent alone or as a mixture with water. Suitable hydrophilic solvents include methanol, ethanol and acetone. The temperature for extraction is usually 0 to 100°C, preferably 5 to 50°C. Extraction may be carried out continuously or batchwise. In the case of batch extraction, the time for extraction is about one hour to 10 days, and the amount of the solvent is usually 1 to 30 times by weight, preferably 3 to 10 times by weight based on the dry material. Extraction may be carried out either by dipping or by stirring. The insoluble residue is removed by, for example, filtration from the extract obtained in the above manner, followed by purification. For purification, known methods for the separation and purification of crude drug components can be employed. For example, it is preferred to employ the two-phase solvent partitioning method, column chromatography, preparative high-performance liquid chromatography, in combination.

The two-phase solvent partitioning methods include, for example, a method in which proanthocyanidin is collected into the solvent phase by partition between a solvent such as n-butanol or methyl ethyl ketone and water, and a method in which proanthocyanidin is collected into the aqueous phase by partition between diethyl ether and water. Column chromatography includes a method using an adsorption resin such as Diaion HP-20 or Sepabeads SP-207, and gel filtration using Sephadex LH-20 or the like. They are employed alone or in combination, if necessary repeatedly.

Preparative high-performance liquid chromatography includes a reversed phase method using octadecyl silica (ODS) column or the like, and a normal phase method using silica gel column or the like.

The purification method to be employed can be arbitrarily selected from the above purification methods and other known purification methods depending upon the starting materials for extraction, the desired purification degree, etc. The desired proanthocyanidin in which the extent of galloylation per constitutive monomer is 10% or lower (molar ratio) can be obtained by employing them, if necessary repeatedly or in combination, or further in combination with treatment with tannase.

The cosmetic composition as described herein may comprise, as may be required, components usually employed in cosmetics or agents for external application in addition to the above essential component.

The additional components include solid oils, semisolid oils, liquid oils, moisturizers, emollients, surfactants, water-soluble high molecular substances, oil-soluble high molecular substances, organic and inorganic pigments, organic powders, ultraviolet absorbents, antiseptics, antioxidants, pH regulators, microbicides, vitamins, crude drugs, crude drug components, skin softeners, perfumes, pigments, ethanol and purified water. They can be added in such range of quality and quantity that the object and effect of the pharmaceutical described herein is not impaired.

Examples of the solid oils and semisolid oils are Vaseline, lanolin, ceresin, microcrystalline wax, carnauba wax, candelilla wax; higher fatty acids such as coconut oil fatty acid, lauric acid and hardened beef tallow fatty acid; and higher alcohols such as lauryl alcohol, cetyl alcohol, stearyl alcohol and behenyl alcohol.

Examples of the liquid oils are vegetable oils such as avocado oil, olive oil and jojoba oil; fatty acids such as oleic acid and isostearic acid; alcohols such as hexadecyl alcohol and oleyl alcohol; ester oils such as cetyl 2-ethylhexanoate, 2-octyldodecyl myristate, neopentylglycol di-2-ethylhexanoate, glycerol tri-2-ethylhexanoate, 2-octyldodecyl oleate, isopropyl myristate, glycerol triisostearate and diglyceride 2-ethylhexanoate; ester oils such as octyldodecyl long-chain acylglutamates; silicone oils such as dimethyl polysiloxane, methyl hydrogen polysiloxane, methylphenyl polysiloxane and octamethyl cyclotetrasiloxane; and liquid hydrocarbon oils such as liquid paraffin squalene and squalane.

The moisturizers include fat-soluble moisturizers, low molecular moisturizers and high molecular moisturizers.

Examples of the fat-soluble moisturizers are lysolecithin, lecithin, cholesterol, cholesterol esters and sphingolipids.

Examples of the low molecular moisturizers are serine, glutamine, sorbitol, mannitol, glycerin, sodium pyrrolidonecarboxylate, 1,3-butylene glycol, propylene glycol, lactic acid and lactates.

Examples of the high molecular moisturizers are hyaluronic acid, sodium hyaluronate, elastin, alginic acid, mucopolysaccharides, polyethylene glycol, polyaspartates, and water-soluble chitin.

Examples of the emollients are cholesteryl long-chain acylglutamates, cholesteryl hydroxystearate, 12-hydroxystearic acid, stearic acid, rhodinic acid and lanolin fatty acid cholesteryl ester.

Examples of the surfactants are nonionic surfactants such as POE cetyl ether, POE stearate, POE sorbitan monolaurate, glycerin fatty acid esters, polyglycerin fatty acid esters and polyoxyethylene hardened castor oil; cationic surfactants such as benzalkonium chloride, stearyltrimethylammonium chloride, dicetyldimethylammonium chloride and behenyltrimethylammonium chloride; amphoteric surfactants such as 2-cocoyl-N-carboxymethyl-N-hydroxyethylimidazolinium betaine and betaine amido acetate; and anionic surfactants such as higher alcohol sulfates, higher alcohol ether sulfates, long-chain fatty acid alkali metal salts, long-chain fatty acid alkaline earth metal salts, long-chain fatty acid basic amino acid salts, N-long-chain acylamino acids and N-long-chain acylamino acid salts. These surfactants are available from Nikko Chemicals Co., Ltd., Nihon Emulsion Co., Ltd., etc.

Examples of the water-soluble high molecular substances are those generally employed in cosmetics such as carboxymethyl cellulose, methyl cellulose, hydroxymethyl cellulose, polyvinyl alcohol, polyvinylpyrrolidone, tragacanth

gum, carrageenan, dextrin, dextrin fatty acid esters, carboxyvinyl polymers, xanthane gum, gelatin, sodium alginate and gum arabic.

Examples of the oil-soluble high molecular substances are those generally employed in cosmetics such as polyvinylpyrrolidone-eicosene copolymer, polyvinylpyrrolidone-hexadecene copolymer, nitrocellulose and high molecular silicone.

Examples of the organic and inorganic pigments are inorganic pigments such as silicic acid, silicic acid anhydride, magnesium silicate, talc, sericite, mica, kaolin, iron oxide red, clay, bentonite, titanium-coated mica, bismuth oxychloride, zirconium oxide, magnesium oxide, zinc oxide, titanium oxide, aluminium oxide, calcium sulfate, barium sulfate, magnesium sulfate, calcium carbonate, magnesium carbonate, iron oxide, ultramarine blue, chromium oxide, chromium hydroxide, carmine, carbon black, and complexes thereof; organic pigments such as polyamide, polyester, polypropylene, polystyrene, polyurethane, a vinyl resin, a urea resin, a phenol resin, a fluororesin, a silicone resin, an acrylic resin, a melamine resin, an epoxy resin, a polycarbonate resin, divinyl benzene-styrene copolymer, silk powder, cellulose, CI pigment yellow and CI pigment orange; and complexes of these inorganic pigments and organic pigments.

Examples of the organic powders are metal soaps such as calcium stearate; alkylphosphoric acid polyvalent metal salts such as sodium zinc cetylphosphate, zinc laurylphosphate and calcium laurylphosphate; acylamino acid polyvalent metal salts such as an N-lauroyl-β-alanine calcium salt, an N-lauroyl-β-alanine zinc salt and an N-lauroylglycine calcium salt; amidosulfonic acid polyvalent metal salts such as an N-lauroyl-taurine calcium salt and an N-palmitoyltaurine calcium salt; N-acyl basic amino acids such as Nε-lauroyl-L-lysine, Nε-palmitoyllysine, Nα-palmitoylornithine, Nα-lauroylarginine and Nα-hardened beef tallow fatty acid acylarginine; N-acylpolypeptides such as N-lauroylglycylglycine; α-amino fatty acids such as α-aminocaprylic acid and α-aminolauric acid; and resin powders such as polyethylene, polypropylene, nylon, polymethyl methacrylate, polystyrene, divinylbenzene-styrene copolymer and ethylene tetrafluoride.

Examples of the ultraviolet absorbents are para-aminobenzoic acid derivatives such as para-aminobenzoic acid and octyl para-dimethylaminobenzoate; benzophenone derivatives such as 2-hydroxy-4-methoxybenzophenone and dihydroxydimethoxybenzophenone; methoxycinnamic acid derivatives such as ethyl para-methoxycinnamate and octyl para-methoxycinnamate; salicylic acid derivatives such as octyl salicylate and homomenthyl salicylate; α-dehydro amino acid derivatives such as N-benzoyl-O-methyl-α-dehydrotyrosine 2-ethylhexyl ester; benzalhydantoin derivatives such as 4-(3,4-dimethoxyphenyl)methylene-2,5-dioxo-1-imidazolidinepropionic acid 2-ethylhexyl ester; urocanic acid, ethyl urocanate, 4-(tert-butyl)-4'-methoxydibenzoylmethane and 2-(2'-hydroxy-5'-methylphenyl)benzotriazol.

Examples of the antiseptics are methylparaben, propylparaben, isopropylparaben and sorbic acid.

Examples of the antioxidants are butylhydroxyanisole, gallic acid, propyl gallate and erythorbic acid.

Examples of the pH regulators are citric acid, lactic acid, arginine, triethanolamine, sodium hydroxide and potassium hydroxide.

Examples of the microbicides are hinokitiol, triclosan, chlorhexidine gluconate, phenoxyethanol, resorcin, isopropylmethylphenol, azulene, salicylic acid and zinc pyrithione.

Examples of the vitamins are dl-α-tocopherol acetate, dl-α-tocopherol, vitamin E, nicotinamide, D-pantothenyl alcohol, biotin and riboflavin.

Examples of the crude drugs and components thereof are aloe extract, ginseng extract and dipotassium glycyrrhizinate.

Examples of the skin softeners are liquid paraffin, Vaseline, olive oil, squalane, lanolin and synthetic ester oils.

As the perfume or pigment, any of those usually employed in cosmetics can be employed.

Any of the above components can be added in such range that the object and effect of the present invention are not impaired. It is preferably 0.001 to 5% (w/w), more preferably 0.01 to 3% (w/w).

There is no specific restriction as to the forms of the cosmectic composition described herein. For example, they can take the form of solution, emulsion or paste mixture.

The cosmetic composition described herein are in the form of a lotion, enriched lotion, emulsion, pack, moisturizing cream, sun-screening cream, massage cream, hand cream, after-shaving cream, body lotion, cleansing cream, cleansing milk, cleansing lotion, cleansing foam or foundation; pharmaceutical preparations such as ointment, cream, or liquid preparations for external application; or the like.

The effective content of proanthocyanidin in the cosmetic compositions described herein is 0.001 to 20% (w/w), preferably 0.01 to 10% (w/w).

The present invention is described in more detail referring to the following reference examples, test examples and examples.

Reference Example 1

Process for Producing Proanthocyanidin Wherein the Proportion of Ungalloylated Proanthocyanidin oligomers Which Are Dimers or Trimers to the Total Proanthocyanidin is 40% or Higher (w/w) (Compound 1)

Commercially available apple juice (21.6 liters) was passed through a column (9 cm ∅ x 50 cm: 3179 ml volume) packed with Diaion HP-20 resin (Mitsubishi Kasei Corporation) equilibrated with water, and the column was washed with 9 liters of desalted water and 2 liters of methanol. The desired substance was eluted with 1 liter of methanol, and the eluate was concentrated under reduced pressure and then passed through a column (7.2 cm ∅ x 48 cm: 1953 ml volume) packed with Diaion HP-20 resin (Mitsubishi Kasei Corporation) equilibrated with water. After the column was washed successively with 4 liters of desalted water, 4 liters of 20% (v/v) aqueous methanol and 4 liters of 30% (v/v) aqueous methanol, the desired substance was eluted with 4 liters of 40% (v/v) aqueous methanol. Then, the solvent was evaporated under reduced pressure to obtain 6.1 g of a solid. The obtained solid was dissolved in 50 ml of 25% (v/v) aqueous methanol, and the resulting solution was passed through a column (3.4 cm ∅ x 30 cm: 272 ml volume) packed with Sephadex LH-20 equilibrated with 25% (v/v) aqueous methanol. The column was washed successively with 500 ml of 25% (v/v) aqueous methanol and 500 ml of 50% (v/v) aqueous methanol. The desired substance was eluted with 500 ml of 75% (v/v) aqueous methanol and the solvent was evaporated under reduced pressure to obtain 1.5 g of Compound 1. Analysis of Compound 1 by reversed phase chromatography revealed that the contents of procyanidin B-1 [epicatechin-(4β→ 8)-catechin] (PB1), procyanidin B-2 [epicatechin-(4β→8)-epicatechin] (PB2) and procyanidin C-1 [epicatechin-(4β→ 8)-epicatechin-(4β→8)-epicatechin] (PC1) were 8.9%, 25.7% and 9.9%, respectively, based on the total proanthocyanidin, and the content of ungalloylated proanthocyanidin oligomers which are dimers or trimers based on the total proanthocyanidin was more than 40% (w/w).

Reference Example 2

Process for Producing Proanthocyanidin in Which the Extent of Galloylation Per Constitutive Monomer is 25% (Molar Ratio) (Compound 2)

Extraction and purification of proanthocyanidin from seeds of grapes of Chardonnay variety was carried out according to the method of Takahashi, et al. described in Acta Dermato Venereologica, Vol. 78, p. 428-432 (1998) to obtain grape seed-derived proanthocyanidin [Compound 2, average polymerization degree: 3.5, extent of galloylation per proanthocyanidin constitutive monomer: 25% (molar ratio)] [calculation of the average polymerization degree and the extent of galloylation: refer to Phytochemistry, Vol. 36, p. 781-784 (1994)].

Test Example 1 Measurement of Water Content of Mouse Skin

[Test method]

Male 6-week-old SKH1 hairless mice (Charles River, Japan Inc.) were fed for two weeks for adaptation, and Composition 1 obtained in Example 1 and Composition 2 obtained in Example 2 were respectively applied to the whole of the skin on the back of each mouse in an amount of 200 μl once per day. After 15 days, the water content of the skin was measured. To the mice of the control group was applied a composition comprising the same components as the above compositions excluding proanthocyanidin. The number of animals was n=4.

[Evaluation method]

Measurement of the water content of the test parts was made intermittently on the morning (prior to the application of composition). The conductivity (water content) of the skin was measured with SKICON-200 [IBS Co., Ltd.] by putting the probe vertically to the test part of the skin. A high measurement value indicates a high water content. The change in the conductivity of the skin at a test part (relative conductivity) was calculated according to equation 1 as the relative value based on the change in the conductivity at a control part.

[Equation 1]

Relative conductivity (%) = (conductivity at a test part on each day/conductivity at a test part on day 0) / (conductivity at a control part on each day/conductivity at a control part on day 0) x 100

The results are shown in Table 1.

Table 1 Water content of skin horny layer measured 15 days after application of composition

| Test substance | Relative conductivity (%) |
|---|---|
| Control | 102.1 |
| Composition 1 | 160.3 |
| Composition 2 | 53.4 |

There was observed an increase in the water content of the mouse skin treated with Composition 1 comprising ungalloylated proanthocyanidin oligomers which are dimers or trimers in an amount of more than 40% (w/w) based on the total proanthocyanidin. On the other hand, the water content lowered in the mouse skin treated with Composition 2 in which the extent of galloylation per proanthocyanidin constitutive monomer is 25% (molar ratio).

Test Example 2

Measurement of Ceramide Content of Mouse Epidermis

[Test method]

Epidermis was sampled from the mouse skin of Test Example 1 by Dispase treatment, and ceramide was extracted therefrom according to the method of Imokawa, et al. described in Journal of Investigative Dermatology, Vol. 96, p. 523-526 (1991) and was determined.

[Evaluation method]

Epidermis was sampled from the Dispase-treated skin. To the epidermis was added an extraction solvent (hexane:ethanol = 95:5, 20 ml for 30 mg of epidermis), followed by sonication for 20 minutes. After the resulting mixture was filtered through a 0.5 μm filter, the solvent was evaporated under reduced pressure to obtain an extract. The extract was dissolved in chloroform and was analyzed by silica gel thin layer chromatography. As the developing solvent, chloroform/methanol/acetic acid = 190/9/1 was used. After 8% (w/v) phosphoric acid containing 10% (w/v) copper sulfate was sprayed, the silica gel was treated at 180°C for 10 minutes to detect ceramide. The amount of ceramide was determined with a densitometer (CS-9000, Shimadzu Corporation). As controls, ceramide type III (SIGMA) and ceramide type IV (SIGMA) were used. The ceramide content was calculated as the relative value based on that of a part without application of the compositions.

The results are shown in Table 2.

Table 2 Ceramide content of epidermis measured 15 days after application of composition

| Test substance | Ceramide content of epidermis (%) |
|---|---|
| Control | 110.3 |
| Composition 1 | 157.4 |
| Composition 2 | 113.2 |

The ceramide content was increased in the epidermis of mice treated with Composition 1 comprising ungalloylated proanthocyanidin oligomers which are dimers or trimers in an amount of more than 40% (w/w) based on the total proanthocyanidin, and the effect of skin improvement was observed. On the other hand, there was observed no

change in the ceramide content of the epidermis of mice treated with Composition 2 in which the extent of galloylation per proanthocyanidin constitutive monomer is 25% (molar ratio).

Test Example 3

Measurement of Water Content of Human Skin

[Test method]

Three healthy women aged between 24 and 29 were chosen as the panelists, and Composition 3 obtained in Example 3 and Composition 4 obtained in Example 4 were respectively applied to the test parts at the inside of their antebrachia in an amount of 2 $\mu$ 1/cm$^2$ twice per day in the morning and in the evening for one month. To the control parts was applied the same amount of a composition comprising the same components as the above compositions excluding proanthocyanidin.

[Evaluation method]

Evaluation was carried out in a manner similar to that in Test Example 1.
The results are shown in Table 3.

Table 3 Water content of skin

| Test substance | Relative conductivity (%) | | | | | |
|---|---|---|---|---|---|---|
| | day 0 | day 7 | day 10 | day 14 | day 21 | day 24 |
| Composition 3 | 100 | 133 | 150 | 156 | 136 | 143 |
| Composition 4 | 100 | 94 | 119 | 116 | 107 | 101 |

There was observed an increase in the water content at the test parts treated with Composition 3 comprising ungalloylated proanthocyanidin oligomers which are dimers or trimers in an amount of more than 40% (w/w) based on the total proanthocyanidin. On the other hand, there was observed no increase in the water content at the test parts treated with Composition 4 in which the extent of galloylation per proanthocyanidin constitutive monomer is 25% (molar ratio).

Test Example 4 (not part of the invention)

Measurement of Collagen Synthesis-promoting Activity on Human Fibroblasts

[Test method]

Measurement of the amount of synthesized collagen was carried out in the following manner according to the method described in Nagai, et al., Collagen Test Method, p. 201-271, Kodansha Scientific (1986).
Human neonate-derived fibroblasts (Sanko Junyaku Co., Ltd.) were suspended in DMEM containing 10% fetal bovine serum [Virology, 8, 396 (1959)] and cultured at 37°C in 5% $CO_2$ to the stationary phase. To the resulting culture were added $^3$H-labeled glycine and a test substance, followed by further culturing for 72 hours. After 500 $\mu$mol/l phenylmethanesulfonyl fluoride (PMSF) was added to the obtained culture, the cells and the medium were collected, followed by disruption with a sonicator to extract proteins. To the extract were added 100 $\mu$mol/l bovine serum albumin (BSA) and 10% trichloroacetic acid (TCA), and the resulting mixture was centrifuged at 3000 rpm for 5 minutes to collect the precipitate. To the precipitate was added a 0.2 mol/l aqueous solution of sodium hydroxide so that the amount of the solution became 8 $\mu$l per cm$^2$ of the bottom area of the culture vessel used in the above culturing. The resulting solution was neutralized with an equal volume of a 1 mol/l phosphate buffer. To 100 $\mu$l of the obtained protein solution were added 10 $\mu$mol/l of 25 mmol/l calcium chloride, 20 $\mu$mol/l of 62.5 mmol/l N-ethylmaleimide and 20 units of collagenase, and the resulting mixture was subjected to reaction at 37°C for 90 minutes.
After the reaction mixture was separated by ultrafiltration (molecular weight: 10,000), the radioactivity of the protein fraction in the filtrate (CDP) was measured as the amount of newly synthesized collagen. Then, the radioactivity of the protein fraction which was not filtered (NCDP) was measured, and the collagen synthesis ratio was calculated according to equation 2. The relative collagen synthesis-promoting activity (the change in collagen synthesis) was calculated according to equation 3 as the relative value of the collagen synthesis ratio in the presence of a test substance

based on the control.

[Equation 2]

$$\text{Collagen synthesis ratio} = \text{CDP}/(\text{CDP} + \text{NCDP}) \times 100$$

[Equation 3]

$$\text{Relative collagen synthesis-promoting activity (\%)} = \text{collagen synthesis ratio of a test substance}/\text{collagen synthesis ratio of control} \times 100$$

The results are shown in Table 4.

Table 4 Relative collagen synthesis-promoting activity on fibroblasts after 72 hours of culturing

| Test substance | Relative collagen synthesis-promoting activity (%) |
|---|---|
| Control | 100 |
| 300 μmol/l PB2 | 120.8 |

The amount of collagen synthesized was increased by the addition of 300 μmol/l procyanidin B-2 to human fibroblasts as compared with that of the control (=100%).

<u>Test Example 5</u> (not part of the invention)

Measurement of Collagenase-inhibiting Activity on Human Fibroblasts

[Test method]

Measurement of collagenase-inhibiting activity was carried out in the following manner according to the method described in Development and Application of Simple Microassay for Collagenase Activity; Nagai, et al., Inflammation, Vol. 4, p. 123-130, Nihon Igakukan (1984).

To 25 μl of a neutralization buffer (0.1 mol/l Tris hydrochloride, pH 7.5, 0.4 mol/l NaCl, 10 mmol/l $CaCl_2$ and 0.04% $NaN_3$) was added 25 μl of fluorescein isothiocyanate (FITC)-labeled collagen I (Cosmo Bio Co.) To the resulting mixture were further added 50 μl of collagenase I (MMP-1) (Cosmo Bio Co.) and a test sample dissolved in 10 μl of the neutralization buffer, followed by reaction at 37°C for 4 hours. To the reaction mixture was added 5 μl of a reaction terminating solution [80 mmol/l o-phenanthroline/50% (v/v) aqueous ethanol] to stop the reaction. To the resulting mixture was added 100 μl of a twice diluted neutralization buffer and the resulting mixture was allowed to stand at 35°C for 60 minutes. After cooling on ice for 5 minutes, 200 μl of 70% (v/v) aqueous ethanol was added to the mixture, followed by vigorous stirring for 5 minutes. The resulting mixture was centrifuged at 15,000 rpm at 4°C for 10 minutes to obtain the supernatant. By using a spectrophotofluorometer, the supernatant was irradiated with excitation light at 495 nm and emission light at 520 nm (A) was measured to determine the amount of decomposed collagen. The collagenase-inhibiting activity was calculated according to equation 4 by using data (B) obtained when purified water was employed in place of collagenase I and data (C) obtained when a neutralization buffer was employed in place of the test sample.

[Equation 4]

$$\text{Collagenase-inhibiting activity (\%)} = [(A)-(B)]/[(C)-(B)] \times 100$$

The results are shown in Table 5.

Table 5 Collagenase-inhibiting activity

| Test substance | Collagenase-inhibiting activity (%) |
|---|---|
| Control | 0 |
| 1 mmol/l PB2 | 100 |
| 100 μmol/l PB2 | 69.4 |
| 100 μ mol/l PC1 | 100 |
| 10 μ mol/l PC1 | 37 |
| 1 μ mol/l PC1 | 17 |

Each proanthocyanidin exhibited collagenase I-inhibiting activity.

Examples

Example 1

Compound 1 obtained in Reference Example 1 [0.5% (w/w)], 1,3-butylene glycol [5% (w/w)], ethanol [30% (w/w)] and purified water [64.5% (w/w)] were mixed with stirring and the solid material was dissolved to prepare Composition 1.

Example 2

Composition 2 was prepared in a manner similar to that in Example 1 except that Compound 1 obtained in Reference Example 1 was replaced with Compound 2 obtained in Reference Example 2.

Example 3

Compound 1 obtained in Reference Example 1 [0.5% (w/w)], ethanol [10% (w/w)] and purified water [89.5% (w/w)] were mixed with stirring and the solid material was dissolved to prepare Composition 3.

Example 4

Composition 4 was prepared in a manner similar to that in Example 3 except that Compound 1 obtained in Reference Example 1 was replaced with Compound 2 obtained in Reference Example 2.

Example 5 Preparation of a Lotion

| | |
|---|---|
| (Oil phase components) | |
| Perfume | 0.05 g |
| Polyoxyethylene (60 mol) hardened castor oil | 2.0 g |
| 1,3-Butylene glycol | 5.0 g |
| (Aqueous phase components) | |
| Compound 1 | 0.5 g |
| Glycerin | 5.0 g |
| Methylparaben | 0.1 g |
| Citric acid | 0.1 g |
| Sodium citrate | 0.2 g |
| Ethanol | 8.0 g |
| Purified water | Appropriate amount |
| Total amount | 100.0 g |

The oil phase components and the aqueous phase components were respectively made into homogeneous

solutions, and the oil phase was added to the aqueous phase with stirring to obtain a lotion.

Example 6 Preparation of an Emulsion

| | |
|---|---|
| (Oil phase components) | |
| Squalane | . 4.0 g |
| Wheat germ oil | 2.0 g |
| Monoglyceryl stearate | 1.0 g |
| Polyoxyethylene stearyl ether | 4.0 g |
| Propylparaben | 0.1 g |
| (Aqueous phase components) | |
| Compound 1 | 0.5 g |
| Methylparaben | 0.1 g |
| Propylene glycol | 0.1 g |
| Polyethylene glycol 6000 | 0.2 g |
| Purified water | Appropriate amount |
| 1% sodium hyaluronate | 5.0 g |
| Total amount | 100.0 g |

The oil phase components and the aqueous phase components were respectively heated to 80°C to make them homogeneous, and the aqueous phase was added to the oil phase with stirring to obtain an emulsion.

Example 7 Preparation of a Cream

| | |
|---|---|
| (Oil phase components) | |
| Squalane | 5.0 g |
| Olive oil | 3.0 g |
| Hydrogenated lanolin | 2.0 g |
| Beeswax | 2.5 g |
| Glyceryl monostearate | 2.0 g |
| Polyoxyethylene (10 mol) stearyl ether | 2.5 g |
| Sorbitan monostearate | 1.5 g |
| 1,3-Butylene glycol | 5.0 g |
| Perfume | Trace amount |
| (Aqueous phase components) | |
| Glycerin | 5.0 g |
| Carboxyvinyl polymer | 0.03 g |
| Triethanolamine | 0.03 g |
| Compound 1 | 0.5 g |
| Methylparaben | 0.3 g |
| Purified water | Appropriate amount |
| Total amount | 100.0 g |

The oil phase components and the aqueous phase components were respectively heated to 80°C to make them homogeneous, and the aqueous phase was added to the oil phase with stirring. After being emulsified, the mixture was cooled to obtain a cream.

Example 8 Preparation of a Powder

| | |
|---|---|
| Compound 1 | 2.0 g |
| Methylparaben | 0.1 g |
| Gum arabic | 0.5 g |
| Citric acid | 0.1 g |
| Sodium citrate | 0.2 g |
| Mannitol | Appropriate amount |

The components were mixed homogeneously with stirring to obtain a powder.

Example 9 Preparation of an Enriched Lotion

| | |
|---|---|
| (Oil phase components) | |
| Cholesteryl ether | 0.2 g |
| Ether pyroglutamate | 1.0 g |
| Lanolin | 0.3 g |
| 1,3-Butylene glycol | 5.0 g |
| Perfume | 0.04 g |
| (Aqueous phase components) | |
| Compound 1 | 0.5 g |
| Sodium chondroitin sulfate | 0.02 g |
| 1,3-Butylene glycol | 4.0 g |
| Ethanol | 1.0 g |
| Methylparaben | 0.1 g |
| 1% Carbopol 940 | 5.0 g |
| 1% Hyaluronic acid | 8.0 g |
| 0.3% Atelocollagen | 1.0 g |
| Purified water | Appropriate amount |
| Total amount | 100.0 g |

The oil phase components and the aqueous phase components were respectively made into homogeneous solutions, and the oil phase was added to the aqueous phase with stirring to obtain an enriched lotion.

Industrial Applicability

Herein there is provided cosmetic compositions having the functions to increase the water content of the skin and to promote the epidermal ceramide synthesis.

## Claims

1. Cosmetic use of a composition comprising, as an active ingredient, proanthocyanidin in which the extent of galloylation per constitutive monomer is 10 % or lower (molar ratio) for increasing skin water-content or promoting epidermal ceramide synthesis.

2. Use according to Claim 1, wherein the proanthocyanidin is not derived from pine bark.

3. Use according to Claim 1, wherein the proanthocyanidin is derived from apple.

4. Use according to any of Claims 1 to 3, wherein the proanthocyanidin comprises ungalloylated proanthocyanidin oligomers (dimers to 5-mers).

5. Use according to any of Claims 1 to 4, wherein the proanthocyanidin content is 0.01 to 10% (w/w).

6. Use according to any of Claims 1 to 5, wherein the proportion of ungalloylated proanthocyanidin oligomers which are dimers to the total proanthocyanidin is 30% or higher (w/w).

7. Use according to any of Claims 1 to 5, wherein the proportion of ungalloylated proanthocyanidin oligomers which are dimers or trimers to the total proanthocyanidin is 40% or higher (w/w).

8. Use according to any of Claims 1 to 5, wherein the proportion of ungalloylated proanthocyanidin oligomers which are dimers to 5-mers to the total proanthocyanidin is 50% or higher (w/w).

**Patentansprüche**

1. Kosmetische Verwendung einer Zusammensetzung, die als aktiven Inhaltsstoff Proanthocyanidin umfaßt, wobei das Ausmaß an Galloylierung pro konstitutivem Monomer 10% oder weniger (molares Verhältnis) beträgt, zum Erhöhen des Wassergehalts der Haut oder zum Fördern der Synthese von epidermalem Ceramid.

2. Verwendung nach Anspruch 1, wobei das Proanthocyanidin nicht von Pinienrinde abgeleitet ist.

3. Verwendung nach Anspruch 1, wobei das Proanthocyanidin von Apfel abgeleitet ist.

4. Verwendung nach einem der Ansprüche 1 bis 3, wobei das Proanthocyanidin ungalloylierte Proanthocyanidin-Oligomere (Dimere bis 5-mere) umfaßt.

5. Verwendung nach einem der Ansprüche 1 bis 4, wobei der Proanthocyanidingehalt 0,01 bis 10% (w/w) beträgt.

6. Verwendung nach einem der Ansprüche 1 bis 5, wobei der Anteil an ungalloylierten Proanthocyanidin-Oligomeren, die Dimere sind, an der Gesamtmenge von Proanthocyanidin 30% oder mehr (w/w) beträgt.

7. Verwendung nach einem der Ansprüche 1 bis 5, wobei der Anteil an ungalloylierten Proanthocyanidin-Oligomeren, die Dimere oder Trimere sind, an der Gesamtmenge von Proanthocyanidin 40% oder mehr (w/w) beträgt.

8. Verwendung nach einem der Ansprüche 1 bis 5, wobei der Anteil an ungalloylierten Proanthocyanidin-Oligomeren, die Dimere bis 5-mere sind, an der Gesamtmenge von Proanthocyanidin 50% oder mehr (w/w) beträgt.

**Revendications**

1. Utilisation en cosmétique d'une composition comprenant, comme ingrédient actif, de la proanthocyanidine dans laquelle le degré de galloylation par monomère constitutif est de 10 % ou moins (rapport molaire) pour augmenter la teneur en eau de la peau ou pour stimuler la synthèse des céramides épidermiques.

2. Utilisation selon la revendication 1, dans laquelle la proanthocyanidine n'est pas dérivée de l'écorce de pin.

3. Utilisation selon la revendication 1, dans laquelle la proanthocyanidine est dérivée de la pomme.

4. Utilisation selon l'une quelconque des revendications 1 à 3, dans laquelle la proanthocyanidine comprend des oligomères de proanthocyanidine non galloylée (dimères à pentamères).

5. Utilisation selon l'une quelconque des revendications 1 à 4, dans laquelle la teneur en proanthocyanidine est de 0,01 à 10 % (p/p).

6. Utilisation selon l'une quelconque des revendications 1 à 5, dans laquelle la proportion d'oligomères de proanthocyanidine non galloylée, qui sont des dimères, par rapport à la proanthocyanidine totale est de 30 % ou plus (p/p).

7. Utilisation selon l'une quelconque des revendications 1 à 5, dans laquelle la proportion d'oligomères de proanthocyanidine non galloylée, qui sont des dimères ou des trimères, par rapport à la proanthocyanidine totale est de 40

% ou plus (p/p).

**8.** Utilisation selon l'une quelconque des revendications 1 à 5, dans laquelle la proportion d'oligomères de proanthocyanidine non galloylée, qui sont des dimères à des pentamères, par rapport à la proanthocyanidine totale est de 50 % ou plus (p/p).